# EUROPÄISCHE PATENTSCHRIFT

(11) **EP 1 269 976 B1**
(45) Veröffentlichungstag und Bekanntmachung des Hinweises auf die Patenterteilung: **02.09.2009**
(21) Anmeldenummer: 02014196.6
(22) Anmeldetag: 26.06.2002
(51) Int. Cl.: A61K 8/22, A61K 8/35, A61K 8/38, A61Q 5/08

(54) **Mittel zum Blondieren von menschlichen Haaren**
Bleaching agent for human hair
Agent pour blondir les cheveux humains

(30) Priorität: 29.06.2001 DE 10131540
(43) Veröffentlichungstag der Anmeldung: 02.01.2003
(73) Patentinhaber: KPSS-Kao Professional Salon Services GmbH, 64297 Darmstadt (DE)
(72) Erfinder: Nöcker, Bernd, Dr., 64372 Ober-Ramstadt (DE); Wilz, Rüdiger, 64319 Pfungstadt (DE); Ghiasi, Fariba, 60316 Frankfurt (DE); Garbe, Barbara, 64625 Benheim (DE)

(56) Entgegenhaltungen:
- EP-A- 1 059 077
- EP-A- 1 059 078
- EP-A- 1 059 079
- EP-A- 1 059 080
- EP-A- 1 059 081
- EP-A- 1 232 741

## Beschreibung

Die vorliegende Erfindung betrifft ein neues Mittel zum intensiven, jedoch gleichwohl schonenden Blondieren und Aufhellen von menschlichen Haaren.

Das Blondieren menschlicher Haare besteht üblicherweise aus folgenden Verfahrensschritten:
Homogenes Vermischen einer wasserfreien Zubereitung, vorzugsweise eines Pulvers, das mindestens eine Verbindung mit bleichender bzw. aufhellender Wirksamkeit, insbesondere ein festes Peroxidsalz, vorzugsweise Ammonium-, Kalium- und/oder Natriumpersulfat oder Erdalkaliperoxide enthält, mit einer wäßrigen Wasserstoffperoxid-Lösung, Aufbringen dieser Zusammensetzung auf das Haar, und Ausspülen nach vollendeter Blondierung. Dabei ist seit längerem bekannt, daß die Verwendung der im Hinblick auf die Blondierung wirksamsten Bestandteile, insbesondere der Persulfate, vor allem des Ammoniumpersulfats, in den erforderlichen höheren Konzentrationen zu Haarschädigungen führen kann.

Es wurde bereits versucht, dieses Problem durch (Teil-)Substitution der Persulfate zu lösen, was bisher jedoch nicht in zufriedenstellender Weise gelungen ist.

Gegenstand der Erfindung ist ein Blondierpulver gemäß Anspruch 1.

Es wurde nunmehr gefunden, und das ist Gegenstand der vorliegenden Erfindung, daß durch den Zusatz mindestens eines Ubichinons zu wasserfreien, insbesondere Peroxide als Wirkstoff enthaltenden Blondiermitteln überraschenderweise nicht nur eine verbesserte Blondierwirkung erreicht wird, ohne eine wesentliche Haarschädigung hervorzurufen, sondern auch noch die Eigenschaften des so behandelten Haares insbesondere, die Naß- und Trockenkämmbarkeit sowie der Griff und das Volumen verbessert werden.

Gelöst wird diese Aufgabe durch den Zusatz von 0,001 bis 10 Gew.-%, berechnet auf die Gesamtzusammensetzung, mindestens eines Ubichinons der Formel (I) 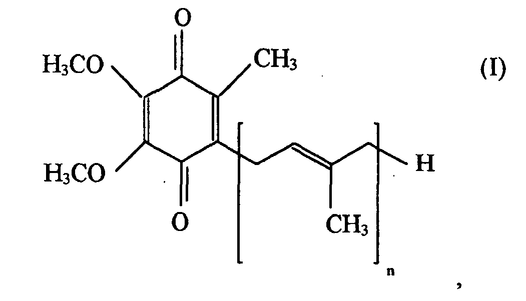 worin n eine Zahl von 1 bis 10 bedeutet.
Bevorzugte Ubichinone sind solche mit n = 6 bis 10, insbesondere Ubichinon 50, worin n 10 bedeutet, auch unter der Bezeichnung "Coenzym Q 10" bekannt.
Die Verwendung von "Coenzym Q 10" in kosmetischen Mitteln, insbesondere in Haarpflegemitteln, ist an sich bekannt.
Die EP 0 751 762 B beschreibt Haarpflegemittel, die ein oder mehrere Ubichinone, insbesondere Coenzym Q 6 und Coenzym Q 10, in Kombination mit Retinolen,
Retinalen und β-Carotin, zur Behandlung der Hautalterung enthalten.
Aus der DE 199 26 167 A1 sind Stylingmittel auf Wasserbasis bekannt, die ein oder mehrere Biochinone wie Ubichinone und Plastochinone zum Schutz der Kopfhaut und des Haares vor unerwünschten Oxidationsprozessen enthalten.
Die DE 199 26 168 A1 offenbart Stylingmittel auf alkoholischer Basis mit Biochinonen, insbesondere Coenzym Q 10.
Die DE 199 26 170 A1 betrifft die Verwendung eines oder mehrerer Biochinone wie Ubichinone in kosmetischen Haarreinigungsmitteln.
Schließlich beschreibt die DE 199 26 156 A1 die Verwendung von Biochinonen wie Ubichinon zur Herstellung von kosmetischen Zubereitungen zur Verbesserung der Haarstruktur, insbesondere der Kämmbarkeit des Haares, also haarkonditionierenden Zusammensetzungen.

Gegenüber diesem Stand der Technik war die verbessernde Bleichwirkung des Zusatzes von Ubichinonen in Blondiermitteln, insbesondere bei Anwendung auf vorgeschädigtem Haar, wobei zusätzlich auch noch dessen kosmetische Eigenschaften wie Glanz, Griff und Sprungkraft verbessert werden, nicht vorhersehbar.
Die bevorzugte Einsatzkonzentration der Coenzyme Q1 bis Q 10 beträgt etwa 0,005 bis 5, insbesondere etwa 0,01 bis 2,5, besonders bevorzugt etwa 0,05 bis 1 Gew.-%, berechnet auf die das Blondierpulver (ohne Oxidationsmittel) enthaltende Zusammensetzung.

Gemäß einer bevorzugten Ausführungsform der Erfindung werden die Blondier- und Aufhellungspulver als staubfreie Produkte verwendet, die als Granulate bzw. Agglomerate vorliegen.

Es kann sich dabei beispielsweise um die in der EP-A 560 088 beschriebenen Produkte, die ein ÖI bzw. ein flüssiges Wachs enthalten, handeln, wobei das Aufbringen dieses Öls oder Wachses zweckmäßigerweise durch Aufsprühen erfolgt.

Auch staubfreie Blondierpulver, die entsprechend der EP-A 630 643 durch Agglomerieren der pulverförmigen Ausgangsbestandteile mittels Aufsprühen von geschmolzenen Wachsen oder C₁₀-C₁₈-Fettsäurealkanolamiden oder Verschmelzen mit denselben bei erhöhter Temperatur hergestellt wurden, sind erfindungsgemäß geeignet.

Neben dem aktiven Peroxid-Bestandteil enthalten die Blondiermittelzusammensetzungen noch die in solchen Mitteln üblichen Bestandteile:
Wenn es sich um ein Pulver handelt, insbesondere inerte pulverförmige Trägerstoffe, sind das beispielsweise pyrogenes Siliciumioxid, Stärkepulver, etc., Alkalisierungsmittel wie Natriummetasilikat, oberflächenaktive Substanzen und
Bindemittel. Zur Vermeidung von Wiederholungen wird auf die einschlägigen Standardwerke, beispielsweise K. Schrader, "Grundlagen und Rezepturen der Kosmetika, 2. Auflage (1989, Hüthig Buchverlag), S.815 bis 823, verwiesen.
Als aktive Bestandteile werden Peroxide eingesetzt. Als solche sind insbesondere Persulfate wie Natrium- und Kaliumpersulfat, Ammoniumpersulfat, Erdalkaliperoxide wie Magnesiumperoxid, Melaminperoxid, Harnstoffperoxid oder Phtholimidoperoxyhexansäure zu nennen.
Der Anteil an Peroxiden liegt bei mindestens 10, vorzugsweise mindestens 20 bis etwa 80 Gew.-%.
Gemäß einer bevorzugten Ausführungsform der Erfindung können die Blondiermittel noch 0,1 bis 10 Gew.-%, berechnet auf die Gesamtzusammensetzung, eines oder mehrerer Ammoniumsalze enthalten.

Geeignete Ammoniumsalze sind dabei Ammoniumcarbonat, Ammoniumhydrogencarbonat, Ammoniumcarbamat, Ammoniumchlorid, Ammoniumsulfat, Ammoniumphosphate, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumartrat, Ammoniumbenzoat, Ammoniumacetat, Ammoniumformiat und Ammoniumlactat.

Bevorzugt sind hierbei die Ammoniumphosphate wie NH₄H₂PO₄, (NH₄)₂HPO₄, (NH₄)₂NaPO₄, NaNH₄HPO₄ oder NH₄Na₂PO₄, Ammoniumchlorid, Ammoniumsulfat und Diammoniumhydrogencitrat sowie Ammoniumcarbonat, vorzugsweise in einer Menge von 0,1 bis 10 Gew.-%.

Die Ammoniumverbindungen können auch, wie aus der EP 609 796 A2 bekannt, in entsprechend höheren Mengen als alleiniger Blondierwirkstoff verwendet werden.

Der Anteil der bleichend bzw. aufhellend wirksamen Verbindungen insgesamt liegt zwischen etwa 5 und etwa 75, insbesondere etwa 25 und etwa 60 Gew.-%, bezogen auf das Aufhellungs- und Blondierpulver.

Die Teilchengrößen der erfindungsgemäßen Blondiermittel liegen in der Regel unterhalb 1 mm, vorzugsweise unterhalb 500 Mikron, beispielsweise bei weniger als 400 Mikron, insbesondere etwa 25 bis etwa 100 µm, was eine ausgezeichnete Verarbeitbarkeit, d.h. Mischbarkeit mit einer wäßrigen Wasserstoffperoxid-Lösung vor Applikation auf das menschliche Haar gewährleistet.

Die Anwendung des Mittels geschieht in an sich bekannter und üblicher Weise:
Das pulverförmige Aufhellungsmittel wird mit einer 6- bis 12-prozentigen Wasserstoffperoxid-Lösung, vorzugsweise in einem Verhältnis von etwa 1 Gewichtsteil des Pulvers zu etwa 0,5 bis etwa 4, insbesondere etwa 1 bis etwa 2,5 Gewichtsteilen der Peroxid-Lösung oder -Lotion homogen vermischt und anschließend etwa 10 bis etwa 60, insbesondere etwa 20 bis 30 Minuten, je nach Temperatur, auf dem Haar zur Einwirkung gebracht.

Die Anwendung kann auch in der Weise geschehen, daß das Blondierpulver und die Wassertoffperoxidlösung mit einer Cremegrundlage vermischt werden, und diese homogene Mischung dann auf das Haar aufgetragen wird.

Vorzugsweise ist die Zusammensetzung so eingestellt, daß beim Mischen mit wäßriger Wasserstoffperoxid-Lösung ein pH-Wert der gebrauchsfertigen Mischung von etwa 8 bis etwa 11,5, insbesondere zwischen 9 bis 11, erhalten wird.

Die folgenden Beispiele dienen der Illustration der Erfindung.

### Beispiel 1

### a) Aufhellungspulver

| | |
|---|---|
| Siliciumdioxid | 16,27 (Gew.-%) |
| Ammoniumchlorid | 14,70 |
| Natriumcarbonat | 10,00 |
| Natriumetasilicat | 2,30 |
| Phthalimidoperoxyhexansäure | 41,50 |
| Natriumpersulfat | 15,20 |
| Coenzym Q6 | 0,03 |

Diese Zusammensetzung wird im Gewichtsverhältnis 1:2,5:2,5 mit einer H₂O₂-Lotion b) und einer Cremegrundlage c) vermischt:

### b) Wasserstoffperoxid-Lotion

| | |
|---|---|
| Wasserstoffperoxid | 6,00 (Gew.-%) |
| Cetylstearylalkohol | 1,70 |
| Phosphorsäure | 0,50 |
| Natriumlaurylsulfat | 0,20 |
| Coenzym Q6 | 0,05 |
| Dinatriumhydrogenphosphat | 0,10 |
| Salicylsäure | 0,10 |
| Wasser | ad 100,00 |

### c) Cremegrundlage

| | |
|---|---|
| Cetylstearylalkohol | 11,0 (Gew.-%) |
| Oleth-5 | 5,0 |
| Ölsäure | 2,5 |
| Stearamide MEA | 2,3 |
| Cocoamide MEA | 2,3 |
| Natriumcetylstearylsulfat | 1,2 |
| 1,2-Propylenglykol | 1,0 |
| 1,2-Propylenglykolstearat | 0,6 |
| Natriumlaurylsulfat | 0,5 |
| Weizenproteinhydrolysat | 0,9 |
| Organopolysiloxan | 0,4 |
| Panthenol | 0,6 |
| Parfum | 0,4 |
| Komplexbildner | 0,2 |
| Wasser | ad 100,0 |

Der pH-Wert dieser Mischung lag bei 9,0.
Bei der Applikation dieser Mischung wurde eine ausgezeichnete, gleichmäßige Blondierwirkung mit guter Naß- und Trockenkämmbarkeit und ausgezeichnetem Glanz festgestellt; eine wesentliche Haarschädigung trat an Haarsträhnen aus ungeschädigtem Menschenhaar nicht auf.
Das Weglassen des "Coenzym Q6" in den Zusammensetzungen führte zu einer verringerten, weniger gleichmäßigen Blondierwirkung, und einem weniger konditionierten Haar.

### Beispiel 2

### Blondierpulver

| | |
|---|---|
| Kieselgur | 2,60 (Gew.-%) |
| Natriumcarboxymethylcelluslose | 2,50 |
| Hydroxyethylcellulose | 1,60 |
| Natriumlaurylsulfat | 2,50 |
| Natriumstearat | 1,60 |
| Eiweißhydrolysat | 0,60 |
| Stärke | 1,00 |
| Natriumcarbonat | 0,60 |
| Natriummetasilikat | 9,00 |
| Polyvinylpyrrolidon K90 | 3,20 |
| Kokosfettsäuremonoethanolamid | 9,80 |
| (NH₄)₂HPO₄ | 2,00 |
| Magnesiumperoxid | 3,80 |
| Kaliumpersulfat | 8,00 |
| Natriumpersulfat | 50,00 |
| Organopolysiloxan | 1,00 |
| Coenzym Q10 | 0,20 |

Es wird ein staubfreies Pulver erhalten, das mit einer bekannten 6%igen H₂O₂-Lösung im Gewichtsverhältnis 1:1 gut angerührt werden kann.
Der pH-Wert dieser Mischung liegt bei 9,0.

Auch bei Anwendung dieser Mischung wurde eine ausgezeichnete, gleichmäßige Blondierwirkung mit guter Naß- und Trockenkämmbarkeit und ausgezeichnetem Glanz festgestellt; eine wesentliche Haarschädigung trat an Haarsträhnen aus ungeschädigtem Menschenhaar nicht auf.
Weglassen des "Coenzym Q10" ergab einen deutlich verringerten Blondiereffekt.

### Beispiel 3

### Staubfreies Blondierpulver

| | |
|---|---|
| Siliciumdioxid (Diatomenerde) | 3,20 (Gew.-%) |
| Siliciumdioxid (pyrogenes SiO₂) | 5,25 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 3,50 |
| Kaliumpersulfat | 57,00 |
| Magnesiumperoxid | 4,00 |
| Organopolysiloxan | 1,00 |
| Coenzym Q10 | 0,05 |
| Paraffinöl (Paraffinum perliquidum, | 11,50 |
| DAB 9) | |

Die Herstellung des Pulvers erfolgte durch Aufsprühen des Paraffinöls auf die Pulvergrundmasse bei etwa 20°C im Wirbelsprühverfahren.
Es wird ein staubfreies Pulver erhalten, das mit einer bekannten 6%igen H₂O₂-Lösung im Gewichtsverhältnis 1:1 gut angerührt werden kann.
99% der Teilchen hatten einen Durchmesser von <400 Mikron.

Die Blondierwirkung und der Glanz des Haares sind ausgezeichnet bei guter Naßund Trockenkämmbarkeit; eine wesentliche Haarschädigung war nicht nachweisbar. Das Weglassen des Coenzym Q10 führte zu einer verringerten und ungleichmäßigeren Blondierwirkung; das Haar wies einen rauheren Griff und eine verringerte Naß- und Trockenkämmbarkeit auf.

### Beispiel 4

### Staubfreies Blondier-Agglomerat

| | |
|---|---|
| Siliciumdioxid (Diatomenerde) | 3,20 (Gew.-%) |
| Siliciumdioxid (pyrogenes SiO₂) | 5,30 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 4,40 |
| Natriumpersulfat | 39,50 |
| Kaliumpersulfat | 14,02 |
| Magnesiumperoxid | 4,00 |
| Cocosmonoethanolamid | 15,00 |
| Coenzym Q10 | 0,05 |
| Coenzym Q6 | 0,03 |

Die Herstellung des Pulvers erfolgte durch Aufheizen der obengenannten Mischung auf 70°C bis 75°C in einem Wirbelstromgenerator und anschließendes Abkühlen.
Es wurde ein staubfreies Pulver enthalten, das mit einer bekannten 9%igen H₂O₂-Lösung im Gewichtsverhältnis 1:1,5 gut angerührt werden konnte. 99% der Teilchen hatten einen Durchmesser von <400 Mikron.
Die am Haar erzielte Blondierwirkung war gleichmäßig ausgezeichnet bei guter Naßund Trockenkämmbarkeit des Haares; eine wesentliche Haarschädigung war nicht erkennbar.
Das Weglassen der Coenzyme Q10 und Q6 führte zu einer verringerten Konditionier- und Blondierwirkung.

### Beispiel 5

### Staubfreies Blondierpulver

| | |
|---|---|
| Pyrogenes Silicia (Aerosil) | 4,00 (Gew.-Teile) |
| Hydroxyethylcellulose | 1,70 |
| Polyvinylpyrrolidon | 4,00 |
| Tetranatrium-EDTA | 2,00 |
| Natriumstearat | 1,25 |
| Natriumcarbonat | 1,00 |
| Ammoniumpersulfat | 8,00 |
| Magnesiumperoxid | 10,00 |
| Kaliumpersulfat | 44,00 |
| Natriummetasilikat | 11,50 |
| Natriumlaurylsulfat | 0,50 |
| Organopolysiloxan-Pfropfcopolymerisat | 1,00 |
| Coenzym Q10 | 0,05 |
| Aufgesprühtes Paraffinöl | 11,00 |

Das Pulver wurde mit einer wäßrigen 6%-igen H₂O₂-Zusammensetzung im Verhältnis 1:1 gemischt und die Mischung auf das Haar aufgebracht. Nach erfolgter Einwirkung, Waschen und Trocknen wurde ein gut und gleichmäßig blondiertes Haar mit dezentem Glanz, weichem Griff und guter Naß- und Trockenkämmbarkeit erhalten.

Das Weglassen der Coenzyme Q10 führte zu einer verringerten Konditionier- und Blondierwirkung.

## Patentansprüche

1. Wasserfreies Pulver zum Blondieren von menschlichen Haaren, enthaltend
a) mindestens eine Verbindung mit bleichender bzw. aufhellender Wirkung, in einer Menge von 5 bis 75 Gew.-%, bezogen auf das Blondierpulver und
b) 0,001 bis 10 Gew.-%, bezogen auf die Gesamtzusammensetzung, mindestens eines Ubichinons der Formel (I) 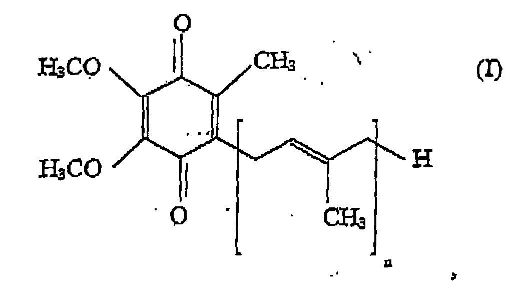
worin n eine Zahl von 1 bis 10 bedeutet.

2. Pulver nach Anspruch 1, worin 10 bedeutet

3. Pulver nach Anspruch 1, worin n 6 bedeutet.

4. Pulver nach einem oder mehreren der Ansprüche 1 bis 3, enthaltend 0,1 bis 2,5 Gew.-% mindestens eines Ubichinons.

5. Pulver nach einem oder mehreren der Ansprüche 1 bis 4, enthaltend als Verbindung mit bleichender bzw. aufhellender Wirkung mindestens ein Peroxid.

6. Pulver nach einem oder mehreren der Ansprüche bis 5, enthaltend als Verbindung mit bleichender bzw. aufhellender Wirkung mindestens ein Ammoniumsalz.

## Claims

1. Water-free powder for the bleaching of human hair, comprising
a) at least one compound with a bleaching or brightening effect in an amount of 5 to 75 % by weight, calculated to the bleaching powder, and
b) 0.001 % to 10 %, calculated to the total composition, of at least one ubiquinone of the formula (I) 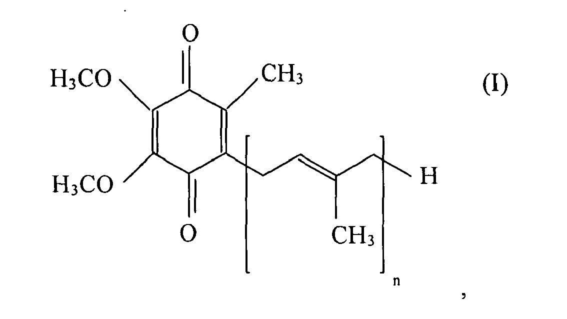
wherein n is a number from 1 to 10.

2. Powder according to claim 1, wherein n is 10.

3. Powder according to claim 1, wherein n is 6.

4. Powder according to one or more of claims 1 to 3, comprising 0.1 % to 2.5 % by weight, calculated to the total composition, of at least one ubiquinone.

5. Powder according to one or more of claims 1 to 4, comprising as compound with a bleaching or brightening effect at least one peroxide.

6. Powder according to one or more of claims 1 to 5, comprising as compound with a bleaching or brightening effect at least one ammonium salt.

## Revendications

1. Poudre sans eau pour la décoloration du cheveu humain, comprenant
a) au moins un composé ayant un effet de décoloration ou d'éclaircissement en une quantité allant de 5 à 75 % en poids, calculé par rapport à la poudre de décoloration, et
b) 0,001 % à 10 %, calculé par rapport à la composition totale, d'au moins une ubiquinone de formule (I) 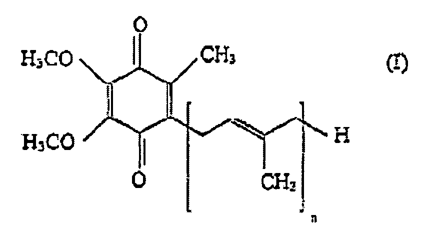 dans laquelle n est un nombre valant 1 à 10.

2. Poudre selon la revendication 1, dans laquelle n vaut 10.

3. Poudre selon la revendication 1, dans laquelle n vaut 6.

4. Poudre selon une ou plusieurs des revendications 1 à 3, comprenant 0,1 % à 2,5 % en poids, calculé par rapport à la composition totale, d'au moins une ubiquinone.

5. Poudre selon une ou plusieurs des revendications 1 à 4, comprenant, comme composé ayant un effet de décoloration ou d'éclaircissement, au moins un peroxyde.

6. Poudre selon une ou plusieurs des revendications 1 à 5, comprenant, comme composé ayant un effet de décoloration ou d'éclaircissement, au moins un sel d'ammonium.
